# EUROPEAN PATENT SPECIFICATION

(11) **EP 3 890 816 B1**
(45) Date of publication and mention of the grant of the patent: **08.04.2026**
(21) Application number: 19893637.9
(22) Date of filing: 06.12.2019
(51) Int. Cl.: A61M 25/00, A61B 10/04

(54) **TUBE-CONTAINING MEDICAL DEVICES WITH BIOACTIVE LUMINAL WIRE**
ROHRHALTIGE MEDIZINISCHE VORRICHTUNGEN MIT BIOAKTIVEM LUMINALEM DRAHT
DISPOSITIFS MÉDICAUX CONTENANT UN TUBE AVEC FIL LUMINAL BIOACTIF

(30) Priority: 06.12.2018 US 201862776408 P
(43) Date of publication of application: 13.10.2021
(73) Proprietor: CathPro Technologies LLC, Salt Lake City, UT 84105 (US)
(72) Inventor: Thiagarajan, Giridhar, Salt Lake City, UT 84105 (US)
(74) Representative: Forresters IP LLP
(86) International application number: PCT/US2019/065075
(87) International publication number: WO 2020/118257

(56) References cited:
- EP-A1- 3 102 277
- WO-A2-03/002020
- WO-A2-2004/062458
- CN-A- 108 853 689
- US-A- 5 180 375
- US-A1- 2006 058 737
- US-A1- 2006 204 738
- US-A1- 2011 092 955
- US-A1- 2014 228 781
- US-A1- 2018 161 546

## Description

### BACKGROUND

Clinicians struggle to manage catheter related blood stream infections (CRBSIs) and luminal occlusions on a daily basis. The occurrence rate of CRBSIs post implementation of maximum barrier precautions and other insertion site protection measures (regular site cleaning, antimicrobial dressings/discs, etc.) has been reduced, but is still in the range of 0.5-2 catheter days per 1,000 catheters, which is based on the number of infections (x) per 1,000 days of insertion, leading to mortality and morbidity. It also causes an economic burden to the healthcare system. CRBSIs can be an issue with long term catheters, where the intraluminal route of infection dominates. Likewise, about 20% of all catheter lumens occlude during implanted life. The predominant cause of catheter occlusions is thrombosis and/or related factors. When lumens occlude, functionality of the catheter becomes limited, as the lumen can no longer be used effectively or at all to aspirate and/or infuse fluids therethrough. Furthermore, drug eluting coatings on devices of various sizes can deplete quickly due to limited availability of the reservoir of drug and non-eluting passive coatings can biofoul, thus restricting the antimicrobial, anti-thrombogenic, and/or thrombolytic effects. For example, there are devices that can deliver bioactive agent of some type into the lumen of a catheter and/or into the body of the subject for a period of a few seconds, a few minutes, a few hours, or even a few days or more. However, some of these catheters and longer term catheters, on the other hand, can be harder to manage, and in many cases, catheters are removed and fully replaced to deal with infection, thrombotic occlusion, etc., putting the subject/patient at further risk. Likewise, replaceable lock solutions have been devised to address longer term catheter complications, but are slow to be developed and marketed due to lengthy clinical trials. In the case of thrombolytic locks, Cathflo^{®} from Genentech (USA), which is a tissue plasminogen activator is available on the market and is effective, but a single fluidic dose is currently well over $100, which is quite expensive considering the considerably lower cost of the catheter hardware itself. US 2006/058737 A1 discloses an indwelling catheter comprising a therapeutic agent carrying stylet.

### BRIEF DESCRIPTION OF THE DRAWINGS

FIG. 1 illustrates an example of a catheter assembly including a luminal wire that can be replaceable in the lumen of a catheter to provide, for example, long-term effectiveness by providing antimicrobial, anti-thrombogenic, thrombolytic, and/or other properties to a lumen of the catheter in accordance with the present disclosure.
FIG. 2 illustrates an example catheter with a Y-junction and multiple hubs having a luminal wire positioned therein that can be replaceable and can be effective for providing antimicrobial, anti-thrombogenic, or thrombolytic, and/or other properties to a lumen of the catheter in accordance with the present disclosure.
FIG. 3 is a graph illustrating the antimicrobial effectiveness of various types of luminal wires, including unmodified metal luminal wire and copper-plated luminal wire, compared to a commercial product in accordance with the present disclosure.
FIGS. 4-5 illustrate a schematic example catheter assembly or kit used in generating data for examples of the present disclosure.
FIG. 6 illustrates a flow system used to simulate blood flow in the human body in generating data for examples of the present disclosure.
FIGS. 7-9 are graphs illustrating lumen patency assessments that were conducted in accordance with examples of the present disclosure.
FIG. 10 illustrates a schematic example catheter assembly or kit used in generating data for examples of the present disclosure.
FIGS. 11-14 provide data for multiple antimicrobial studies related to the use of copper wire inserts in accordance with examples of the present disclosure.
FIGS. 15-16 provide data related to the health and safety of blood when exposed to the copper luminal wires in accordance with examples of the present disclosure.

### DETAILED DESCRIPTION

In a first aspect of the present invention there is provided a tube-containing medical device kit as set forth in the claims. In a second aspect of the present invention there is provided a method of making the tube-containing medical device kit of the invention as set forth in the claims. The present disclosure is drawn to tube-containing medical devices with replaceable bioactive luminal wires, bioactive luminal wires for insertion into a lumen of a tube-containing medical device, and methods of making the tube-containing medical devices with bioactive luminal wires. These devices, systems, and methods can be effective for preventing or reducing intraluminal infection and/or luminal thrombotic occlusion in catheters and other elongated tubular structures of medical devices susceptible to infection, e.g., feeding tubes, tracheal tubes, shunts, drainage tubes, e.g., external ventricular drain (EVD), etc. These systems, devices, and methods can provide for not only short term solutions to the introduction of infection into the lumen of tube-containing medical devices and connectors, but can also exhibit long term effectiveness. To illustrate by way of specific example, one can consider catheters generally. Thus, examples herein drawn specifically to catheters should be read to include other similar tubing examples where the medical device is not a catheter *per se*, e.g., a shunt, drainage tube, trachea, feeding tube, etc. For example, a shunt effectively has two ports, one at each end that redirects fluids within the body from one location to another. In this instance, a hub may be placed outside of the body for insertion of a luminal wire in one or both directions toward the shunt openings, etc. With that understanding, for simplicity in explanation here and elsewhere in the specification, catheters are described in greater detail providing a proxy example of one of the multiple types of medical devices that can be used, and such disclosure is directly relevant and supports other tube-containing medical devices.

With specific reference to catheters, this type of medical device typically includes a hub outside of the body of the patient that can be accessed by a medical professionals or that can be self-accessed by the patient. The hub can be a connector of any type, as defined herein. Catheters can also include a port, which is the structure
that connects the catheter to the body, e.g., to a vein, to a bladder, or to any other body structure the catheter is designed to be inserted. For example, hubs and ports can have an elongated lumen therebetween, and these three structures, e.g., the hub, the elongated tubing defining a lumen, and the port, are often used to access or draw fluids from the body, to introduce locking fluids into the lumen of the catheter, or to introduce therapeutic fluids into the body through the catheter. With respect to longer term antimicrobial, anti-thrombogenic, and/or thrombolytic activity within the catheter lumen, or for general catheter maintenance for even shorter term use, a separate bioactive luminal wire device can be configured to be introduced, or can be pre-positioned within, the lumen of a catheter, and can be replaceable by removing the luminal wire and replacing it with the same type or different type of luminal wire.

Thus, in accordance with an example of the present disclosure, a catheter assembly can include a catheter including an access opening positionable exterior with respect to a subject, a fluid communication opening positionable within the subject to fluidically communicate with a body fluid of the subject, and an elongated lumen therebetween. The catheter assembly can also include a luminal wire inserted in the elongated lumen, wherein the luminal wire has a length that is at least 5% of a length of the elongated lumen, and/or has an x-y cross-sectional diameter sufficiently smaller than an x-y cross-sectional diameter of the elongated lumen to allow insertion and retraction of wire, and furthermore, may also be configured to provide fluid flow therebetween in instances where there may be a desire to flow fluid thereby. The luminal wire can be bioactive, e.g., antimicrobial, anti-thrombogenic, thrombolytic, or can be used to deliver a bioactive agent such as a drug or other therapeutic compound. In one example, if the wire length is longer than 100% of the luminal length (or in some cases even if shorter), there can be a mechanism or other control system in place to prevent the luminal wire from extending past the fluid communication opening, or alternatively indicate to the user where to stop inserting the luminal wire, for example. The luminal wire can also be replaceable with a new luminal wire by simply removing the luminal wire and inserting a replacement luminal wire. In one example, a fluid lock solution can be loaded in the elongated lumen along with the luminal wire.

In another example, a catheter kit can include a catheter including an access opening positionable exterior with respect to a subject, a fluid communication opening positionable within the subject to fluidically communicate with a body fluid of the subject, and an elongated lumen therebetween. The catheter kit can also include a luminal wire insertable within the elongated lumen, wherein the luminal wire has a length that is at least 5% of a length of the elongated lumen, and/or has an x-y cross-sectional diameter sufficiently smaller than an x-y cross-sectional diameter of the elongated lumen to allow insertion and retraction of wire, and furthermore, in some examples, provide fluid flow therebetween in instances where there may be a desire to flow fluid thereby. As a note, space for providing fluid flow can typically be less than that practical for removing and inserting luminal wire, but this is not always the case. In one example, if the wire length is longer than 100% of the luminal length (or in some cases even if shorter), there can be a mechanism or other control system in place to prevent the luminal wire from extending past the fluid communication opening, or alternatively indicate to the user where to stop inserting the luminal wire, for example. The luminal wire can also be replaceable with a new luminal wire by simply removing the luminal wire and inserting a replacement luminal wire. In some types of medical tubing or catheters, the fluid communication opening may be on a side wall of the catheter. In such instances, in one example, the luminal wire can be inserted through the lumen of the catheter to the tip, past the side wall opening, without luminal wire exiting the catheter and entering the patient. Regardless of the arrangement, the luminal wire can be inserted into the lumen of the catheter, in one example, to a length that provides antimicrobial, anti-thrombogenic, and/or thrombolytic properties, and can reside as an indwelling sub-device within the tubing of the catheter or other tubular medical device, at any portion or all of the lumen of the catheter, while preventing the luminal wire from entering the body of the subject outside of the protection of the walls of the tubing.

In another example, a method of manufacturing a catheter assembly or kit can include obtaining a catheter including an access opening positionable exterior with respect to a subject, a fluid communication opening positionable within the subject to fluidically communicate with a body fluid of the subject, and an elongated lumen therebetween. The method can further include establishing a length of a luminal wire that is positionable within at least 5% the elongated lumen, wherein the luminal wire has an x-y cross-sectional diameter sufficiently smaller than an x-y cross-sectional diameter of the elongated lumen to allow removal and/or insertion of a luminal wire. The luminal wire in this example is bioactive. The method can also include sizing the luminal wire to correspond with the length. Sizing can be shortened by cutting, lengthened by stretching in the z-direction of the wire, forming luminal wire at the length so that shortening or lengthening is not required. With this method, the length can be such that the luminal wire is not long enough or otherwise not insertable into the elongated lumen far enough to extend beyond the fluid communication opening. The catheter assembly or kit can further be used to prepare the catheter assembly by inserting the luminal wire in the elongated lumen. The catheter kit can include, for example, multiple interchangeable luminal wires of the same length. Furthermore, the multiple interchangeable luminal wires include a first luminal wire pre-inserted in the elongated lumen, and a second luminal wire as part of the catheter assembly or kit as a replacement luminal wire.

In another example, a method of protecting the lumen of a catheter can include inserting a fluid communication opening of a catheter within a body of a subject, the catheter further including an access opening exterior to the body to access an elongated lumen of the catheter in fluid communication with the fluid communication opening. The method further includes sliding a luminal wire within the elongated lumen, wherein the luminal wire has a length that is within 50% to 100% of the elongated lumen, and has an x-y cross-sectional diameter sufficiently smaller than an x-y cross-sectional diameter of the elongated lumen to allow fluid flow therebetween, and wherein the luminal wire is bioactive. In one example, the step of sliding the luminal wire within the elongated lumen occurs prior to inserting the fluid communication opening of the catheter within the body of the subject. In another example, the step of sliding the luminal wire within the elongated lumen occurs after inserting the fluid communication opening of the catheter within the body of the subject. In one specific example, the method can include applying an electrical potential to the luminal wire, which may be used to provide any of a number of bioactive effects, including improving or enhancing the antimicrobial properties of the luminal wire, such as to improve longevity or to improve antimicrobial efficacy compared to when there is no electrical potential applied.

In addition to the examples described above, e.g., the tube-containing medical device assemblies, the tube-containing medical device kits, the methods of manufacturing, and methods of protecting the lumen of a catheter, certain features will be discussed in greater detail below. It is noted that when discussing the catheter assembly, the catheter kit, or any of the methods herein, these relative details can be considered applicable to the other examples, whether or not they are explicitly discussed in the context of that example. Thus, for example, in discussing a luminal wire related to the catheter assembly, such disclosure is also relevant to and directly supported in the context of the catheter kit and/or methods described herein, and *vice versa,* etc.

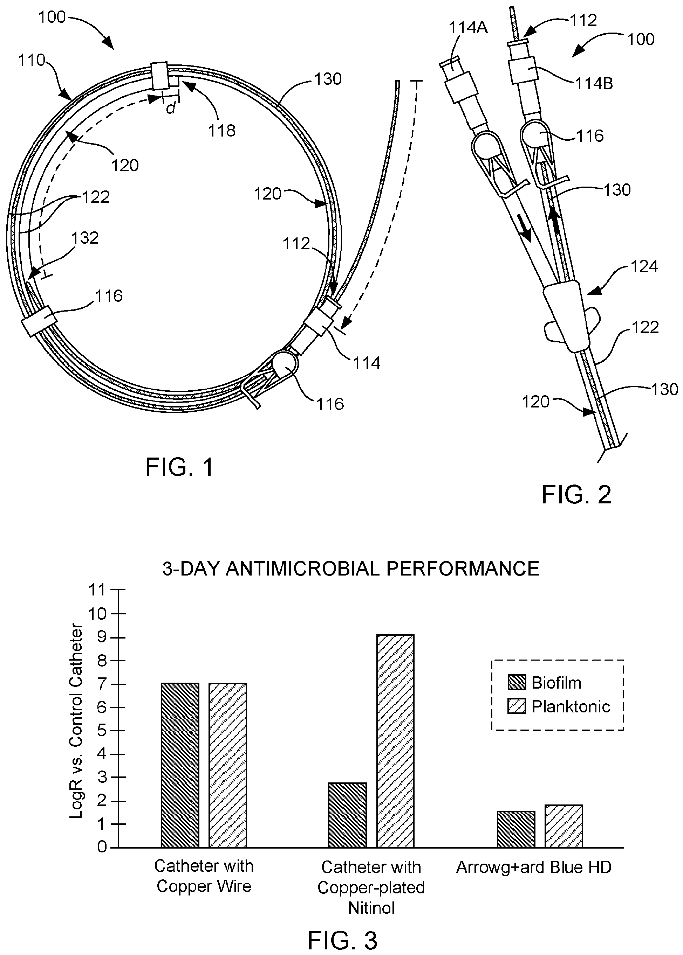

As a point of initial orientation, FIGS. 1 and 2 are provided to show several features of a catheter assembly 100. These FIGS. are also relevant to catheter kits and methods herein, but are shown in the context of the catheter assembly for convenience and to show the spatial relationship of the luminal wire and the catheter in connection to one another during or after assembly. Thus, in these FIGS., the catheter assembly includes a catheter 110 and a luminal wire 130. The catheter in this example includes an access opening 112 defined by a hub 114. The hub can be integrated as part of the catheter or can be assembleable with the tubing. In this particular example, there is also a clamp 116 associated with the catheter near the hub. The catheter also includes a fluid communication opening 118, which in some examples can be defined by or fluidly associated with a port (not shown), e.g., needle port. If present, however, the port may be integrated with the tubing or can be assembleable with the tubing, for example. The port can be, for example, merely a harder polymeric portion of the tubing suitable for insertion of the catheter through the skin and into a vein or artery of a subject (or some other fluidic channel), or can be some other device other than a needle port. Between the access opening and the fluid communication opening is an elongated lumen 120 defined by catheter walls 122 (or the tubing). In one example. The catheter can be branched, as shown generally at 124 in FIG. 2, with multiple hubs 114A, 114B at a Y-junction, for example. The luminal wire 130 shown in FIG. 1 is sized so that when fully inserted, a distal tip 132 of the wire will be positioned just short of the catheter port 118, shown in this example as distance *"d."* In this specific instance, the luminal wire can be about 99% to as long as the elongated lumen 120, though other lengths can be used with other distances short of the fluid communication opening (or port) for example. In further detail, the tube-containing medical device can include an insert hub opening (or wire insertion/removal opening) for inserting or removing the luminal wire from the tubing that is separate from a fluidic hub used for introducing or removing fluid from the tubing. This can provide the benefit of allowing for insertion and/or removal of the luminal wire without occupying or using up a hub opening that may be used for a different reason, or it may be that there is reason to have a separate insertion hub opening that is independent of convenience, e.g., adding an insertion hub to a shunt so that the multiple port ends can be accessed for inserting and/or removing luminal wire in either direction or both directions.

Alternatively, the wire could be attached to a cap (not shown) which provides the same function of preventing the luminal wire from exiting the fluid communication opening at the distal tip if the design of the catheter is such that the wire could exit through an end opening or even side openings in some examples. Thus, the luminal wire can be inserted (attached at the proximal end to the cap) and secured such that the wire is not long enough to pass through the vascular insertion end, or fluid communication opening as previously described, when capped off. Alternatively, an atraumatic tip can also be used for the luminal wire to provide that no damage to the vessel wall or lungs occurs if the wire is accidentally inserted beyond an opening at or near a distal tip (fluid communication opening) of the catheter. Alternatively, a ball tip (which some needles include) could likewise be implemented here. Alternatively, the luminal wire can be equipped with a polymer bit fabricated at its distal tip for additional safety as well.

Turning now to further detail regarding the catheter assemblies, catheter kits, and methods, the luminal wire can be made of a material or coated with a material that is antimicrobial, anti-thrombogenic, and/or provides thrombolytic activity, so that an outer surface of the luminal wire contacts fluid as it flows through the catheter in either direction. Thus, a cross-sectional x-y dimension of the luminal wire is smaller than a luminal surface of the catheter so that the luminal wire can be removed and inserted, for example, and in cases where there may be fluid flow through the catheter or other tubular medical device, the wire can be configured such that fluid can flow between the tubing wall and the luminal wire. Furthermore, if or when the luminal wire loses its therapeutic effect (antimicrobial, anti-thrombogenic, and/or thrombolytic, etc.), the luminal wire can be replaced, for example, while the catheter remains fluidly coupled to the patient, e.g., the catheter does not need to be removed or in some instances even disturbed as an old or spent luminal wire is removed and replaced with a fresh luminal wire insert. By including a replaceable luminal wire, this can eliminate a reservoir effect of bioactive agent, as the luminal wire can be designed to last for a predetermined period of time, e.g., from 1 hour to 12 months, from 1 day to 4 months, from 2 days to 2 months, from 3 days to 1 month, from 1 day to 1 week, etc., and then replaced easily without removing the catheter from the body of the subject. Thus, if a bioactive agent is loaded on a wire substrate, then when the bioactive agent is spent or released, the luminal wire can be replaced with a fresh luminal wire with more bioactive agent or another bioactive agent or with a luminal wire that does not have a separate bioactive agent thereon. This can continue until the life of the catheter is finished, providing a modular mechanism to treat intraluminal microbial colonization and thrombotic occlusions.

The term "luminal wire" herein includes any rigid or semi-rigid device with an antimicrobial, anti-thrombogenic, and/or thrombolytic surface that is elongated in the z-dimension and having a cross-sectional area or size in the x-y-dimension thin or small enough to fit within the lumen of a catheter while retaining enough space between the outer surface of the luminal wire and the surface of the catheter lumen to allow for insertion or removal of the luminal wire, and allow for fluid flow in instances where there may be fluid flow. The term "luminal wire" is thus defined to include a number of structures that are x-y-dimensionally thin and z-dimensionally elongated, such as metal wires, metal alloy wires, multiple wires braided or twisted together, coated wires, tubular wire, multilayered wire, e.g., multiple material in an annular arrangement, composited wire, e.g., multiple wires composited or bound together, etc. For example, a tubular or annular wire arrangement can include a hollow elongated core (or even discrete core pockets present at specific locations, periodically placed, or both) with a metal or metal alloy shell, which can include pores or includes an orifice at an end of the luminal wire that may release bioactive agent loaded within the hollow core. The luminal wire can be flexible and/or malleable so that it can bend or otherwise conform with bending and/or twisting of the catheter tubing, but may be more laterally (x-y-dimension) rigid than the catheter tubing, less laterally rigid than the catheter tubing, or about the same degree of lateral rigidity as the catheter tubing. The bending can be such that the luminal wire conforms to the new shapes and retains that shape, or the bending can be resilient so that after bending or flexing, the luminal wire returns or partially returns to the original orientation prior to bending or flexing with the catheter.

With respect to the luminal wire length, a number of lengths can be used. For example, the luminal wire can have a length that is at least 5% of a length of the elongated lumen, at least 5% of a length of the elongated lumen, at least 10% of a length of the elongated lumen, at least 20% of a length of the elongated lumen, at least 35% of a length of the elongated lumen, or at least 50% of a length of the elongated lumen, and/or can have an x-y cross-sectional diameter sufficiently smaller than an x-y cross-sectional diameter of the elongated lumen to allow insertion and retraction of wire. In one example, the space between the luminal wire and the lumen walls can provide for allowing fluid flow therebetween in instances where there may be a desire to flow fluid thereby. Luminal wire length may or may not alternatively or additionally be limited, for example, by the length of the luminal cavity of the catheter (which can include the catheter tubing defining an elongated catheter lumen, hubs, ports, etc.) or catheter system (which can include other devices, tubing, etc., that may be connectable to the catheter). In other words, whatever portion that should be protected by the luminal wire, the wire can be inserted therein to remain for carrying out its function, e.g., to protect from infection and/or thrombotic occlusion. In some instances, the luminal wire length can terminate at about the distal tip of the catheter within the body of the subject (at or near the port), and in other instances, a distal tip of the luminal wire length can terminate short of a fluid communication opening within the body of the subject, e.g., the port or other opening near the distal end of the catheter. For example, the luminal wire length can be inserted into the catheter through a hub and slid into the catheter until the luminal wire is no longer exposed at the hub end of the catheter, or the wire can include a marking on the luminal wire so that the user (medical practitioner) knows where to stop inserting the wire where in one example, a clamp could be used to prevent further insertion. **In** another example, a mechanical stopper could be associated with the luminal wire to prevent insertion of the luminal wire beyond a certain fixed location, e.g., stopped by the architecture or shape of the hub. In these three examples, the luminal wire can be slid into the catheter and stopped a safe distance prior to insertion of the wire into the body of the subject beyond the protective sheath of the catheter walls. For example, when fully inserted, a distal tip of the luminal wire can be configured to stop just short of a port or other opening of the catheter where fluids communicate between the body of the patient and the lumen of the catheter. If not inserting all the way to the end of the catheter at the distal end, suitable distances where the distal tip of the luminal wire can be positioned just short of a fluid communication opening can be from from 0.1 cm to 55 cm, 0.1 cm to 50 cm, from 0.1 cm to 35 cm, 0.1 cm to 20 cm, from 0.1 cm to 10 cm, 0.1 cm to 5 cm, 0.5 cm to 50 cm, 0.5 cm to 35 cm, 0.5 cm to 20 cm, from 0.5 cm to 10 cm, 0.5 cm to 5 cm, from 1 cm to 20 cm, from 1 cm to 10 cm, or from 1 cm to 5 cm short of the fluid communication opening. By stopping insertion of the luminal wire short of an opening of the catheter (or other tubular medical device with the body of the subject, the luminal wire does not enter the body of the subject outside of the lumen of the catheter, with some margin of safety. In catheters with a fluid communication opening along a side wall of the tubing, the luminal wire can go beyond the fluid communication opening without entering the body of the subject, as there may still be tubing between the fluid communication opening and the distal tip of the catheter within the body of the subject. Either way, the luminal wire can be inserted in a way that remains within the lumen of the catheter/tubing so that the wire cannot cause any damage to tissue within the body of the subject, as it is protected by the walls of the tubing.

In accordance with this, in one example, the luminal wire can extend all the way through the elongated lumen of the catheter, or through 5% to 100%, 10% to 100%, 20% to 100%, 20% to 99.9%, 20% to 99%, 20% to 95%, 20% to 75%, 35% to 100%, 35% to 99.9%, 35% to 99%, 35% to 95%, 35% to 75%, 50% to 100%, 50% to 99.9%, 50% to 99%, 50% to 95%, 50% to 75%, 65% to 100%, 65% to 99.9%, 65% to 99%, 65% to 95%, 65% to 75%, etc., of the elongated lumen, for example. These values are based on how much of the elongated lumen includes luminal wire, not how long the luminal wire *per* se is, as in some examples, the luminal wire can extend out beyond the hub (outside or through the hub), into or through the hub connector, into another tube or device, etc., for example. Thus, the luminal wire may be (in total length), for example, from 5% to 300%, from 10% to 300%, from 5% to 200%, from 10% to 200%, from 5% to 100% from 10% to 100%, from 5% to 99.9%, from 10% to 99.9%, from 5% to 95%, from 10% to 95%, from 20% to 300%, 20% to 300%, 20% to 150%, 20% to 100%, 20% to 99.9%, 20% to 99%, 20% to 95%, 20% to 75%, 35% to 200%, 35% to 150%, 35% to 100%, 35% to 99.9%, 35% to 99%, 35% to 95%, 35% to 75%, 50% to 200%, 50% to 150%, 50% to 100%, 50% to 99.9%, 50% to 99%, 50% to 95%, 50% to 75%, 65% to 200%, 65% to 150%, 65% to 100%, 65% to 99.9%, 65% to 99%, 65% to 95%, 65% to 75%, etc., of the length of the elongated lumen. In one example, if the wire length is longer than 100% of the luminal length (or in some cases even if shorter), there can be a mechanism or other control system in place to prevent the luminal wire from extending past the fluid communication opening, or alternatively indicate to the user where to stop inserting the luminal wire, for example. If the fluid communication opening is on a side wall within the body of the patient (or to be placed within the body), then the luminal wire can be inserted beyond the opening, as the distal tip without a luminal opening can act to stop the luminal wire from being inserted further into the body of the subject. In other examples, the luminal wire can terminate at one end at about the access opening of the hub, e.g., within about 5 cm or about 10 cm on either side of the port opening. At the port, or at an opening along the lumen of the catheter that will be or is inserted within a body of a subject, the wire can terminate at the port or other opening, e.g., within 20 cm, within 10 cm, or within 5 cm short of the port or other opening as described above.

The term "elongated" when referring to the z-dimension of the luminal wire can be defined as having z-dimension length to x-y-dimension cross-sectional diameter ratio of at least 50:1, at least 100:1, at least 200:1, or at least 500:1. Example ratio ranges can be from 50:1 to 3,000:1, from 50:1 to 2,000:1, from 50:1 to 1,000:1, from 50:1 to 500:1, from 100:1 to 3,000:1, from 100:1 to 2,000:1, from 100:1 to 1,000:1, from 100:1 to 500:1, from 200:1 to 3,000:1, from 200:1 to 2,000:1, from 200:1 to 1,000:1, from 200:1 to 500:1, from 500:1 to 3,000:1, from 500:1 to 2,000:1, or from 500:1 to 1,000:1. For example, using the American Wire Gauge (AWG) standard which relates specifically to wire diameter of round/solid wire, a 16 gauge luminal wire that is about 5 feet long may have an aspect ratio of about 1:1,200 or so. An 18 gauge luminal wire that is three feet long may have an aspect ratio of about 1:900 or so. A 12 gauge luminal wire that is about 1 foot long may have an aspect ratio of about 1:150 or so. A 20 gauge luminal wire that is about 6 feet long may have an aspect ratio of about 1:2,250 or so. A table of wire gauges and diameters in inches and millimeters is provided in Table 1 below, but this list or the use of the AWG system is not considered limiting, but rather merely exemplary. Additionally, Table 1 provides relative cross-sectional areas in the x-y-dimension, which would be relevant to luminal wires (including the wire-like structures defined as "luminal wires" herein) that may or may not have a circular aspect ratio.

**Table 1**

| Gauge (AWG) | Diameter (inch) | Area (inch²) | Diameter (mm) | Area (mm²) |
|---|---|---|---|---|
| 6 | 0.1620 | 0.0206 | 4.115 | 13.3 |
| 7 | 0.1443 | 0.0163 | 3.665 | 10.5 |
| 8 | 0.1285 | 0.0130 | 3.264 | 8.37 |
| 9 | 0.1144 | 0.0103 | 2.906 | 6.63 |
| 10 | 0.1019 | 0.0082 | 2.588 | 5.26 |
| 11 | 0.0907 | 0.0065 | 2.305 | 4.17 |
| 12 | 0.0808 | 0.0051 | 2.053 | 3.31 |
| 13 | 0.0720 | 0.0041 | 1.828 | 2.62 |
| 14 | 0.0641 | 0.0032 | 1.628 | 2.08 |
| 15 | 0.0571 | 0.0026 | 1.450 | 1.65 |
| 16 | 0.0508 | 0.0020 | 1.291 | 1.31 |
| 17 | 0.0453 | 0.0016 | 1.150 | 1.04 |
| 18 | 0.0403 | 0.0013 | 1.024 | 0.823 |
| 19 | 0.0359 | 0.0010 | 0.912 | 0.653 |
| 20 | 0.0320 | 0.0008 | 0.812 | 0.518 |
| 21 | 0.0285 | 0.0006 | 0.723 | 0.410 |
| 22 | 0.0253 | 0.0005 | 0.644 | 0.326 |
| 23 | 0.0226 | 0.0004 | 0.573 | 0.258 |
| 24 | 0.0201 | 0.0003 | 0.511 | 0.205 |

In further detail, the cross-sectional area of the luminal wire and the cross-sectional area of the elongated lumen of the tubing, e.g., catheter tubing, can also be established. For example, the luminal wire can have an x-y-dimension cross-sectional area and the elongated lumen (space defined by inner surface of tubing wall(s)) has an x-y-dimension cross-sectional area. Thus, the x-y-dimension cross-sectional area of the luminal wire can occupy an average of 7% to 90% of the x-y-dimension cross-sectional area elongated lumen when the luminal wire is fully inserted in the elongated lumen. Alternatively, the x-y-dimension cross-sectional area of the luminal wire can occupy an average of 25% to 85%, 35% to 75%, 30% to 75%, or from 50% to 90% of the x-y-dimension cross-sectional area elongated lumen when the luminal wire is fully inserted in the elongated lumen.

Regarding the luminal wire material that can provide antimicrobial, anti-thrombogenic, and/or thrombolytic activity within the lumen of the catheter, the luminal wire itself can be of a material that provides one or more of these properties without modification, or the luminal wire can comprise a wire substrate that is coated or composited with an antimicrobial agent, an anti-thrombogenic agent, or a thrombolytic agent. Examples of materials that can be used for the luminal wire (coated or uncoated) include metals or metal alloys, such as copper, silver, zinc, gold, tin, alloys thereof. The term "alloys" includes copper, silver, zinc, and/or gold together, but can also include alloys of one or more of these metals with any other metal(s) or non-metal(s) that may provide a therapeutic or other practical property. For example, as copper oxidizes, copper can be alloyed with another metal, such as tin, zinc, gold, silver, etc., to slow or prevent oxidation. As copper is a good metal for providing antimicrobial properties, the alloy can include a substantial portion of copper and a lesser proportion of other metals (or non-metals) that may be included to slow oxidation, but may not contribute to the antimicrobial properties of the luminal wire to the extent that copper can contribute. Thus, if a copper alloy is used, the copper can be present in the alloy at from 50 wt% to 99 wt%, from 50 wt% to 95 wt%, from 50 wt% to 90 wt%, from 50 wt% to 80 wt%, 55 wt% to 99 wt%, from 55 wt% to 95 wt%, from 55 wt% to 90 wt%, from 55 wt% to 80 wt%, 60 wt% to 99 wt%, from 60 wt% to 95 wt%, from 60 wt% to 90 wt%, from 60 wt% to 80 wt%, or from 55 wt% to 70 wt%.

With more specific reference to copper, in addition to the cationic nature of copper ions (Cu²⁺) that cause them to bind or become attracted to negatively charged protective cell components and obliterate or otherwise disrupt or damage the cell membrane or wall, e.g., bacteria or other microbes, copper can also kill microbes by contact with the elemental metal (rather than by ions that slowly diffuse into solution over time). Thus, copper can act as a contact killer of various microbial organisms. Thus, copper in particular can be a good material for use as the luminal wire *per se* (or as the wire substrate to be coated with a bioactive agent) of the present disclosure, either as an uncoated luminal wire or as a coated or partially coated wire substrate, because it can kill pathogens continuously upon surface contact therewith to actively reduce microbial colonization. This can occur by virtue of the charge density around the interface where the microbe contacts the copper, which may cause membrane damage, nucleic acid damage, and/or generation of a reactive oxygen species that may be detrimental to the microbe. Thus, copper ions do not necessarily need to diffuse or leach out from the surface of the luminal wire and into the fluid to kill microbes in order to have a good antimicrobial effect. Instead, the native elemental or alloyed copper surface can possess active antimicrobial properties that can prevent colonization, and furthermore, the diffusion of ions out into the fluid can enhance further the antimicrobial effect. Because of the contact killing nature of copper in particular, this allows elemental or alloyed copper luminal wire to last long-term without depletion or consumption of the killing effectiveness of the luminal wire. In still further detail, an additional benefit of using a luminal wire with contact killing properties, e.g., copper, is that it may not expose the subject to undesired high dosages of copper. Furthermore, the divalent nature of copper can be particularly effective at killing microbes, compared to monovalent metals, for example.

With respect to metal or metal alloy luminal wires (or metal or metal alloy coatings thereof), apart from the capacity of metal or metal alloy material, such as copper, copper alloy, etc., to effect antimicrobial activity, the wire also can be activated by passing a small electrical potential along or across the luminal wire to produce a more potent effect in terms of distance of efficacy and strength of antimicrobial performance.

As mentioned, the luminal wire can be wire substrate modified with an antimicrobial, anti-thrombogenic, and/or thrombolytic compound that may be composited with, coated on, chemically attached to, etc., a surface of the wire substrate, for example. The coating, if applied, can be included along the entire length of the wire substrate at a surface thereof, along 75% to less than 100% of the wire substrate length, along 50% to less than 75% of the wire substrate length, along 25% to less than 50% of the wire substrate length, or along 0.1% to less than 25% of the wire substrate length. For example, an antimicrobial copper (or other) wire substrate can include a heparin (or other anti-thrombogenic) coating along a length of the wire substrate at or near a distal tip of the wire substrate, which may also be at or near a distal tip opening or other opening of the catheter positioned or positionable within the body of the subject. To illustrate, heparin can be coated from the distal tip of the wire substrate up to about 20 cm from the distal tip, up to about 10 cm from the distal tip, or up to about 5 cm from the distal tip, for example.

Thus, if the luminal wire is a coated or otherwise modified wire substrate with associated antimicrobial agent, anti-thrombogenic agent, thrombolytic agent, drug, or other bioactive agent not inherent in the metal itself, the "luminal wire" can be defined to include a wire substrate, e.g., the metal or alloy of any wire-like configuration such as solid wire, braided wires, twisted wires, composited wires, etc., and the bioactive agent can be associated physically or chemically with the wire substrate to be released therefrom into a catheter fluid or released by fluid passing through the catheter. The bioactive agent can be an antimicrobial agent such as an antibiotic compound, an antifungal compound, an antiviral compound, an anti-thrombogenic compound, a thrombolytic compound, etc., for example. This bioactive agent can thus be a separate component than the material of the wire substrate that may also be antimicrobial, anti-thrombogenic, thrombolytic, etc. If the bioactive agent is an antibiotic, antifungal, or antiviral compound, the bioactive agent associated with the wire substrate can include, for example, vancomycin, clindamycin, rifampin, minocycline, amoxicillin, tetracycline, chlorhexidine, iodine, silver, copper, zinc, gold, curcumin and its derivatives, gentamycin, cephalosporin, etc., or a combination thereof. If the bioactive agent is an anti-thrombogenic compound, the bioactive agent associated with the wire substrate can include, for example, heparin, direct thrombin inhibitors, Factor Xa inhibitors, aspirin, EDTA, citrate, platelet glycoprotein IIb inhibitors, platelet glycoprotein Illa inhibitors, antiplatelet agents, direct P2YR inhibitors, Nitric oxide and precursors, etc., or a combination thereof. If the bioactive agent is a thrombolytic compound, the bioactive agent associated with the wire substrate can include, for example, a tissue plasminogen activator, urokinase, streptokinase, plasmin, etc.

The application of the bioactive agent(s) can be part of a matrix that includes, for example, polymers, sugars, carriers, excipients, or binders, etc. Based on dosing or purpose of the bioactive agent, from a portion of the wire substrate to the entire length of the wire substrate can be physically or chemically associated therewith. In one example, the bioactive agent can be coated on a surface of the wire substrate as part of a coating composition or matrix that may adhere to a surface of wire substrate for immediate release, or which may be released over a time frame that is somewhat predictable. If the wire is designed to be both antimicrobial and include a coating for release of a bioactive agent, for example, then more immediate release or partial coating (leaving some wire substrate portions exposed) from the wire substrate may desirable in some circumstances.

In further detail, if the luminal wire is a coated wire substrate, in one example, the wire substrate can be of a material other than a metal or a metal alloy, and the coating can be a metal or a metal alloy that is electrolytically plated thereon. For example, rather than a metal or metal alloy wire substrate, the wire could include a flexible plastic wire substrate and a metal or metal alloy coating applied thereon by electroplating, for example. Or the opposite could be the case, where a metal wire substrate is joined, composited, or coated with a plastic material, such as toward a distal end of the wire substrate. These arrangements could take advantage of the antimicrobial properties of a metal wire or wire coating, such as copper, and the other plastic material (wire substrate or coating) could take advantage of the non-conductive and non-thrombogenic nature of polymers (relative to metals), and/or the plastics or other polymeric materials could be included as they sometimes can be more easily coated with other compounds, e.g., bioactive agents.

Alternatively, the wire substrate could be a nitinol wire substrate coated with a metal or metal alloy applied by electroplating. Nitinol is an alloy of nickel and titanium with about equivalent atomic percentages of nickel and titanium. Nitinol 60, for example, includes about 60 wt% nickel and about 40 wt% titanium; nitinol 55 includes about 55 wt% nickel and about 45 wt% titanium; and so forth. Most medical grade nitinol wire is about equal atomic parts nickel and titanium, but can be varied for providing different properties. Different grades of nitinol wire can be obtained, such as nitinol #1, nitinol #2, nitinol #3, nitinol #4, nitinol #5, nitinol #6, nitinol #7, nitinol #8, and nitinol #9, for example (available from Fort Wayne Metals - USA). Notably, other providers of nitinol wire can also source this material. Of these nitinol wires mentioned above from Fort Wayne Metals, nitinol #1 and nitinol #4-#9 meet the chemistry requirements set forth by ASTM F2063 for use in surgical implants, and may even have a higher purity than how this ASTM defines "medical grade" purity. That stated, since the nitinol wires of the present disclosure would be coated, or partially coated, with another bioactive material, such as copper or another bioactive metal or metal alloy coating, and as the wire would remain sheathed by the catheter while in use, the medical grade properties may not be needed for use in accordance with the present disclosure. Thus, nitinol #2, nitinol #3, or other non-medical grade nitinol wire could be used if coated, e.g., electro-plated, or otherwise associated with an antimicrobial metal or metal alloy and/or with an antimicrobial, anti-thrombogenic, or thrombolytic compound. In still further detail, nitinol wire can be heat-treated as a super-elastic wire, or can be more malleable. When heat treated, the nitinol wire can allow for strain of up to 8% without permanent kinking at body temperature. When the wire is coated with another metal or metal alloy, the rigidity and/or strain may be modified from this value. Nitinol can be used for guidewires, and as a guidewire, and the guidewire can be the wire substrate that can be coated with a bioactive material, such as a bioactive metal or alloy or other compound to provide a bioactive effect as described herein. Likewise, the nitinol may be simply a wire substrate that is not configured as a guide wire, but is merely a wire substrate to receive a bioactive coating thereon. Nickel and titanium are not particularly bioactive in and of themselves, but with a copper or copper alloy coating, for example, their wire substrate functionality or other properties may be leveraged to include a bioactive function, e.g., anti-infective or antimicrobial properties.

In further detail, the luminal wires that include a wire substrate that is modified with a bioactive compound, such as copper or copper alloy, can be a metal, a metal alloy, e.g., nitinol, or a non-metal (or combination metal/alloy and non-metal). Thus, the wire substrate can be merely a wire with no secondary function other than to support the bioactive compound, or can have a secondary function like a guidewire that is associated with the bioactive compound, e.g., guidewire electroplated with an antimicrobial metal, such as copper or a copper alloy. Thus, a medical professional could insert a catheter into a subject using a guidewire within the catheter, and then rather than remove the guidewire, if coated or constructed from an antimicrobial metal or coated with a bioactive agent, for example, the guidewire could remain in the catheter for a period of hours, days, weeks, months, etc., thus providing antimicrobial, anti-thrombogenic, and/or thrombolytic properties to the lumen of the catheter after set in place by the guidewire. Alternatively, in addition to coating wire substrates of any material by electroplating, alternative coating processes that can be used include sputter coating, dip coating, spray coating, or the like. Thus, for example, in the case of nitinol, which has mechanical properties medical practitioners are accustomed to and which are already FDA approved, an advantage of coating such a material with copper or other antimicrobial, anti-thrombogenic, and/or thrombolytic material allows for the leveraging of technology already approved for use while adding the additional benefit(s) described herein.

In another example, regarding the assemblies, kits, methods, and other examples herein, a length of the luminal wire can be tapered, e.g., along a portion of the length of the luminal wire or along the full length of the luminal wire. In another example, the luminal wire can include a proximal end and a distal tip, wherein the luminal wire includes both an antimicrobial agent and anti-thrombogenic agent, wherein the antimicrobial agent is nearer to the proximal end and the anti-thrombogenic agent is nearer the distal tip. In yet another example, the bioactive agent is activatable by a lock solution in use.

In further detail, there are other additional advantages of using the wires inside the lumen of a catheter as described herein over the use of antimicrobial/anti-thrombogenic solutions to provide antimicrobial and/or anti-thrombogenic properties. First, by adding a wire to an existing catheter design, there may be no reason to redesign an existing catheter that is in common use. By designing a wire of appropriate length and diameter, the wire can simply be slid into the catheter from a catheter opening or hub, stopping just short of an area where the wire might otherwise exit an opening within a body of a subject, e.g., at the port or other opening that may be present on a side wall of the catheter. With a wire that does not diffuse significant amount of ionic metal, there could be designs with minimal body fluid exposure, as contact killing could occur mostly within the lumen of the catheter. If bioactive agents are to be released into the body, those bioactive agent delivery profiles could be controlled based on loading levels, loading locations, coating thicknesses, etc. If the coatings are for intraluminal protection only, they may diffuse out into the lumen of the catheter for protecting the catheter, and to avoid introducing those compounds into the body fluids within the subject, the catheter could be aspirated out before subsequent diffusion of fluidic bioactive agents into the body. Furthermore, the wires can be designed for guiding catheters and/or for imaging, for example, and have the added effect of providing antimicrobial, anti-thrombogenic, and/or thrombolytic properties of the guiding and/or imaging device.

It is to be noted that, as used in this specification and the appended claims, the singular forms "a," "an," and "the" include plural referents unless the context clearly dictates otherwise.

As used herein, the term "about" is used to provide flexibility to a numerical range endpoint by providing that a given value may be "a little above" or "a little below" the endpoint. The degree of flexibility of this term can be dictated by the particular variable and would be within the knowledge of those skilled in the art to determine based on experience and the description herein.

The term "catheter" is used herein to refer generally to devices used to provide fluid access to internal body spaces of a subject, either for infusion of a fluid or withdrawal of a body fluid, or both. This includes transcutaneous access as well as access through ducts, tracts, or other passages. These access devices include, without limitation, vascular catheters, venous catheters, arterial catheters, feeding tubes, injection catheters, perfusion catheters, urinary catheters, and shunts, e.g., ventriculoperitoneal (VP) shunts, ventriculoatrial (VA) shunts, lumboperitoneal (LP) shunts, etc. A catheter typically includes a proximal end with an opening, e.g., at a hub (to fluidly connect to other devices), and a distal end, e.g., at a port, which is inserted in the body of a subject for fluid communication with the subject at an opening within the body (at the port or elsewhere within the body). On the other hand, there are many different types of valves, fittings, junctions, connectors, chambers, fluid delivery devices, bags, syringes, fluid metering devices, other tubing, fittings, medical devices, inserts (other than the wire insert), etc., or the like may be attached to the catheter at the hub (or even at the port to supplement the functionality of the port), for example, and thus, a "catheter system" includes any system that includes a catheter connected to another "device(s)," typically at the port or hub. For example, catheters can be connectable to other structures or devices by luer connector, barbed fitting, pressure fittings, etc., or other connectable structure.

The term "wire" includes any elongated structure that can be placed along a partial or full length of tubing, such as catheter tubing. Thus, wires can be defined to include indwelling bioactive inserts that are elongated in the z-direction, for example, but do not have any specific other dimensional requirements with respect to cross-sectional shape (in the direction of the x- and y-axes). The cross-section shape can be, for example, circular, oval, triangular, square, rhomboidal, trapezoidal, other polygonal shapes, e.g., with 5-16 sides, U-channeled, T-shaped, annular (with an open center), etc. Furthermore, the surface of the wire, in some examples, can be textured to provide more surface area for enhancing bioactive function(s), e.g., more surface area for contact killing, e.g., textures can be patterns, roughed surface, ridged, grooved, stepped, etc. Furthermore, along the length of the wire, the wire can be a cross-sectional shape along the entire length, can be tapered at one end or the other or both, can be thinner at areas between multiple ends, etc. The wire(s) of the present disclosure can be inserted in catheters *per se,* or can be inserted in catheter systems, but when inserted in a catheter system, the wire is at least within a lumen of the catheter, but may pass through or partially through other structures, such as fittings, etc. Wires can be included in catheters as manufactured, or can be inserted after the catheter is in place within a subject, or can be removed and replaced after the catheter is in place within the subject. The wire can be inserted into an opening at a proximal end defined as the end of the catheter where the user or medical professional accesses the catheter. When the wire is inserted, it can be inserted in a direction where a tip of the wire is advanced towards a distal end of the catheter defined as the end of the catheter that is found with a body of a subject or patient for communicating fluids between the subject and the lumen of the catheter when the catheter is in use.

The term "diameter" refers to a distance across the x-y cross-sectional dimension of a wire with a circular cross-sectional shape. For wires of shapes with x-y cross-sectional shapes that are not circular, the "diameter" can be calculated by determining the area of the x-y cross-section and reshaping the area to a circular dimension, thus providing a "diameter" dimension that is based on an area of the calculated circular cross-section.

Sizes, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1.0 to 2.0 percent" should be interpreted to include not only the explicitly recited values of about 1.0 percent to about 2.0 percent, but also include individual values and sub-ranges within the indicated range. Thus, included in this numerical range are individual values such as 1.1, 1.3, and 1.5, and sub-ranges such as from 1.3 to 1.7, 1.0 to 1.5, and from 1.4 to 1.9, etc. This same principle applies to ranges reciting only one numerical value. Furthermore, such an interpretation should apply regardless of the breadth of the range or the characteristics being described.

Percentages herein are wt% unless stated otherwise or the context dictates otherwise.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

It is also noted that any of the device features described herein and/or shown in the FIGS. can be combined together in any manner that is not specifically shown or described. For example, it is not the purpose of the present disclosure to put together every possible combination of features in the drawings or description, but rather describe fully the combination of catheters or catheter systems of various types to be combined with luminal wires of various types.

### EXAMPLES

The following illustrates examples of the present disclosure. However, it is to be understood that the following are merely illustrative of the application of the principles of the present disclosure. Numerous modifications and alternative devices, methods, and systems may be devised without departing from the spirit and scope of the present disclosure. The appended claims are intended to cover such modifications and arrangements.

### Example 1 - 3-day Antimicrobial Performance Study of Copper Wire, Copper-Plated Nitinol, and Arrowg+ard Blue HD

An antimicrobial study was conducted to validate the antimicrobial activity of copper wire and copper-plated nitinol wire (nickel titanium alloy wire, e.g. Nitinol 55, Nitinol 60, etc.), when inserted within the lumen of a 14F hemodialysis catheter. These catheters with luminal wire inserts were compared for antimicrobial effectiveness relative to a commercially available antimicrobial hemodialysis catheter product (ARROWg+ard Blue^{®} HD), which is a technology that impregnates the catheter substrate including the inner lumen of the hemodialysis catheter with chlorhexidine acetate and silver sulfadiazine, both of which are known to be effective antimicrobial compounds. An uncoated hemodialysis catheter was also included in the study as a negative control.

More specifically, for this study, an 18 gauge (AGW) copper wire (100 wt% copper) was inserted into the lumen of a 14F hemodialysis catheter along the entire length thereof. Likewise, a copper plated Nitinol wire (copper plated by electroplating) was inserted into the lumen of another 14F hemodialysis catheter. In addition to the catheters with the luminal wire inserts and the comparative ARROWg+ard Blue^{®} HD catheter (from Arrow International Inc., Reading, PA, USA), a fourth control catheter was also inoculated that was not chemically or structurally modified in any way, e.g., no anti-microbial impregnation, no luminal wires, no antimicrobial fluids, etc. All four catheters were exposed to a single saline lock for 2 days, and then to a microbial inoculum for 24 hours at 37 °C. The microbial species used for the study was *Staphylococcus aureus* prepared in a 5% TSB solution for inoculation. After the 24h incubation, microbial counts on the control hemodialysis catheter was determined, and the LogR reduction for the three other catheters was determined based on a comparison to the control. Both biofilm (adherent to luminal wall of the catheter) and planktonic (free floating in lumen) recoveries are represented in the graph provided in FIG. 3.

As can be seen in the data, the log reduction compared to a Control catheter using luminal wire in accordance with the present disclosure outperformed ARROWg+ard Blue^{®} HD significantly with respect to both biofilm and planktonic growth.

### Example 2 - Preparation of Anti-thrombogenic Luminal Wire for Insertion in Catheter Tubing

A full length (the length of the catheter) nitinol (nickel titanium alloy) wire with heparin coating was prepared with the distal tip (10 cm) of the nitinol wire dipped in a heparin solution (HBAC - heparin-benzalkonium chloride) having 2,500 units of activity (I.U.) per mL, and then dried for a period of 2 hours. This process was repeated 5 times. Thus, the nitinol wire retained its original alloy structure along its length, and the 10 cm of the distal-most tip of the nitinol wire was coated with 5 coatings of heparin.

### Example 3 - Lumen Patency Assessment

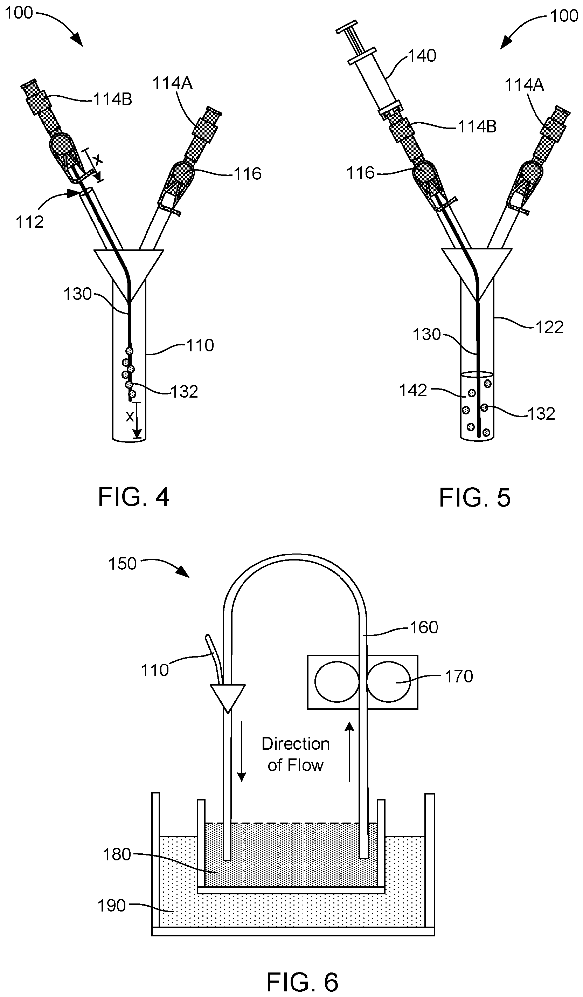

Three separate studies were carried out to determine if the heparin coated luminal wire prepared in accordance with Example 2 provided better lumen patency when inserted in a 7F central venous catheter than a Control catheter (no wire insert or lumen wall coatings). The studies were conducted using a catheter device and insert similar to that shown in FIGS. 4 and 5, and for two of the three studies (Assessment 2 and 3), the blood used in the study was subjected to additional challenges using the flow system shown in FIG. 6 to more closely simulate the fluidics and conditions that may occur with a real patient.

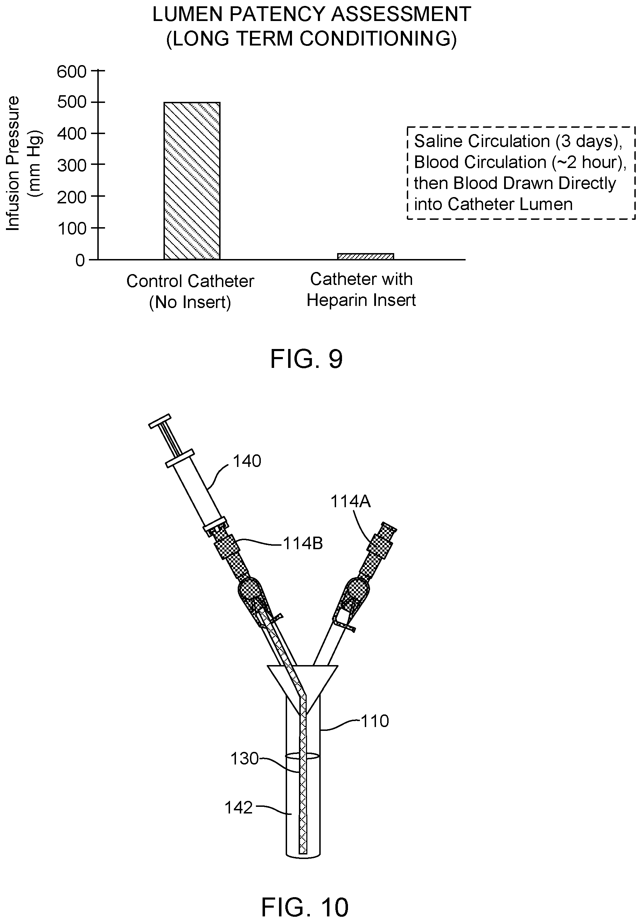

FIGS. 4 and 5 (and FIG. 10) schematically show a device (not to scale) similar to that shown and described in FIGS. 1 and 2, with a catheter assembly 100 including a catheter 110 and a luminal wire 130 (heparin-coated nitinol in FIGS. 4 and 5, and copper wire in FIG. 10). The catheter in this example includes an access opening 112 (or access openings at or near the tip), a pair of hubs 114A, 114B with associated access clamps 116. The luminal wire can be attached to the hub to prevent the distal tip from being inserted beyond the vascular insertion end/opening of the catheter, as shown by distance "x." Alternatively, the wire could be attached to a cap which provides the same function, where the wire is inserted and the cap secured such that the wire is not long enough to pass through the vascular insertion end, or fluid communication opening as previously described, when capped off. With the cap arrangement, blood can be drawn such as with a syringe 140, and then the wire inserted and the end capped off, for example. In this particular example, the distal tip portion of the luminal wire is coated with heparin 132, which when blood 142 is drawn into the lumen of the catheter, the heparin can diffuse into the blood and provide its anti-thrombogenic function.

Regarding FIG. 6, a simulated fluidic device 150 included a fluidic loop of tubing 160, which can be used to circulate saline, blood, or other fluids using a roller pump 170, for example. In examples of the present disclosure, the heparin-coated luminal wire was evaluated in a catheter against a Control catheter without an insert therein. The circulating fluid 180 for this study is kept at about body temperature using a 37 °C water bath.

Assessment 1 was carried out using the catheter 110 and luminal wire insert 130 (coated with heparin) of FIGS. 4 and 5, and Assessments 2 and 3 were carried out using the catheter and insert of FIGS. 4 and 5 as well as the flow system 150 shown in FIG. 6.

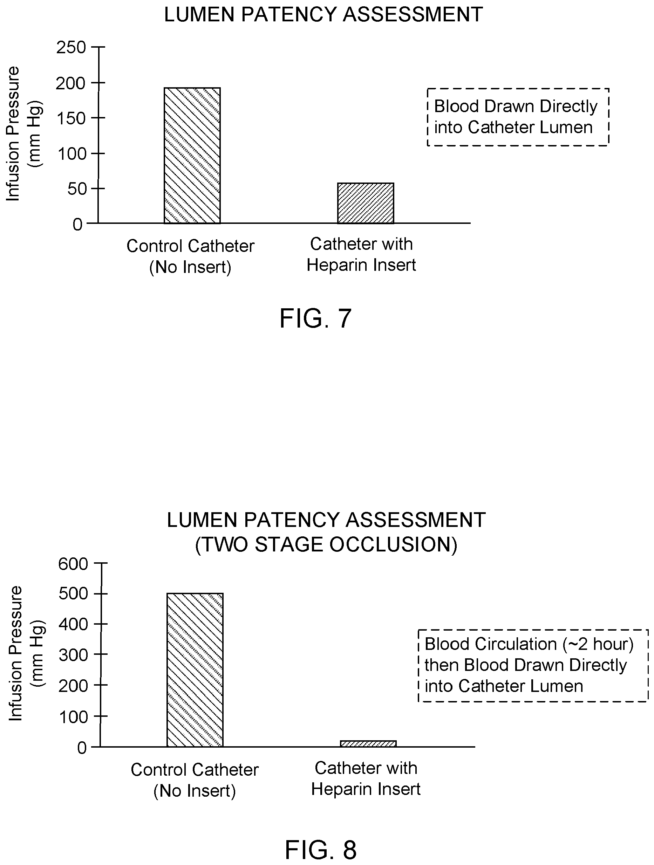

For Assessment 1, blood 142 was drawn into the lumen of the catheter as show in FIG. 5. One catheter included the heparin-coated insert and a Control catheter did not contain the heparin insert (or any other anti-thrombogenic technologies). Once the Control catheter had completely occluded, the infusion pressure using an infusion system was measured from both catheters. Lower infusion pressure compared to the clogged Control catheter indicates that the lumen of the catheter with the heparin insert remains patent. FIG. 7 illustrates the data collected from this assessment. As can be seen, the catheter with the heparin coated wire remained patent at the time that the Control catheter became clogged.

For Assessment 2, a similar evaluation was conducted, but prior to drawing the blood into the two catheters (one with the heparin coated wire insert and a Control catheter with an insert), the blood was circulated for ~2 hour in the flow system followed by drawing the blood into the lumens of the respective catheters. This protocol is more similar to circulating blood in a human subject, e.g., the way thrombosis initiates on a catheter is closer to clinical conditions under human blood circulation. The data collected for this study is provided in FIG. 8, and again, the catheter with the heparin-coated insert remained patent.

For Assessment 3, the same protocol of Assessment 2 was carried out, except prior to the ~2 hour of blood circulation, the tubing of the flow system was circulated with saline for 3 days with the catheter containing heparin-coated wire in place and uncoated Control catheter. This was done to see if the saline would cause the heparin to leach out, rendering the insert ineffective during the subsequent blood challenge. The data collected for this study is shown in FIG. 9. Again, the catheter with the heparin-coated wire insert remained patent when the Control catheter became clogged.

### Example 4 - Antimicrobial Performance of Copper Wire Luminal Inserts

Six separate studies were conducted using various copper wire luminal inserts with 14F hemodialysis catheters compared to various control or comparative devices and/or fluid challenges. The study was conducted using a catheter 110 and luminal wire 130 insert as shown schematically in FIG. 10, which is similar to that shown in FIGS. 1, 2, 4, and 5, except the luminal wire in these examples are made of copper instead of Nitinol or a nitinol wire plating with copper on the outer diameter.

In further detail, for these studies, the following general procedures were followed where applicable. Various lengths (5 cm, 10 cm, 15 cm, 20 cm in one example) of 18 gauge (AGW) copper wire (100 wt% copper) were inserted into the lumen of a 14F hemodialysis catheter (uncoated HD catheters). Control catheters (uncoated hemodialysis catheters) as well as a comparative ARROWg+ard Blue^{®} HD catheter (from Arrow International Inc., Reading, PA, USA), which is a commercial product containing chlorhexidine and silver sulphadiazine coating on the inside and outside, were also evaluated for comparison purposes. In several of these studies, the catheters were exposed to a single saline lock for 2 days, and then to a microbial inoculum for 24 hours at 37 °C. The microbial species used for the study was *Staphylococcus aureus* prepared in a 5% TSB solution for inoculation. After the 24 hour incubation, microbial counts from the different catheters was determined, and the Log reduction for the catheter containing the copper insert and Arrowg+ard Blue^{®} HD was determined based on a comparison to the Control catheter Both biofilm (adherent to luminal wall of the catheter) and planktonic (free floating in lumen) recoveries were collected.

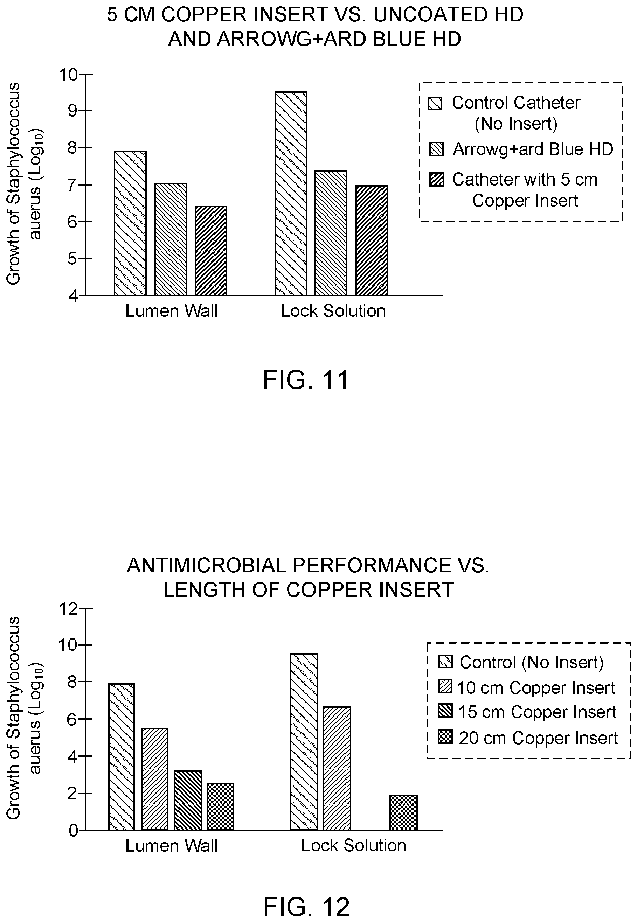

FIG. 11 demonstrates the antimicrobial activity of a very short piece of copper wire (5 cm) compared to the Arrowg+ard Blue HD technology, with the Log₁₀ microbial growth data also provided for the Control catheter (no insert, no coating, hemodialysis catheter). As can be seen in the data collected, the bacterial count with just 5 cm of copper wire had a significantly reduced microbial count compared to the Control catheter, and was comparable or better than the comparative Arrowg+arg Blue HD commercial antimicrobial catheter with respect to the microbial count on the luminal wall count and the lock solution.

FIG. 12 illustrates a comparison of the antimicrobial activity for various lengths of copper wire, e.g., 5 cm, 10 cm, 15 cm, and 20 cm, relative to the Control catheter. As can be seen, as the length of the copper wire is increased, the antimicrobial effect also increased substantially, with the exception that the 15 cm copper wire exhibited better antimicrobial activity than the 20 cm copper wire with respect to microbial recoveries in the lock solution. Nevertheless, the trend of greater lengths of copper wire relative to antimicrobial activity is generally corollary.

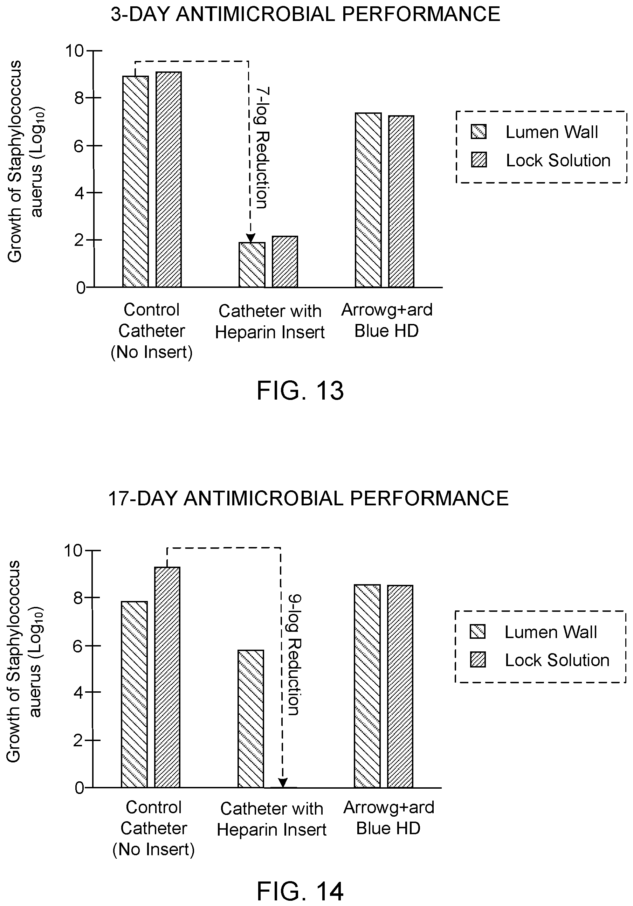

FIG. 13 illustrates the results of a 3-day antimicrobial study similar to that shown and described in Example 1 (FIG. 3). In this instance, however, a copper wire insert of 20 cm in length was evaluated against a Control catheter and the Arrow+ard Blue HD catheter. As can be seen, the copper wire insert outperformed the Arrow+ard Blue HD catheter, exhibit about a 7-log reduction at the luminal wall and the close to that in the lock solution. As a point of reference, a 7-log reduction is a 99.99999% reduction in microbial count relative to the Control catheter. Notably, the data used for this particular graph is from the same study shown and described with respect to Example 1 and FIG. 3, but is presented as microbial growth instead of log reduction so that it can be easily compared to the data collected and present in FIG. 14 (the 17-day study).

FIG. 14 illustrates the results of a 17-day antimicrobial study. In this instance, a copper wire insert of 20 cm in length was evaluated against a Control catheter and the Arrow+ard Blue HD catheter. As can be seen, the copper wire insert outperformed the Arrow+ard Blue HD catheter, exhibit about a 9-log reduction in the lock solution. The luminal wall log reduction was not as impressive but was still significantly better than the performance of the Arrow+ard Blue HD catheter.

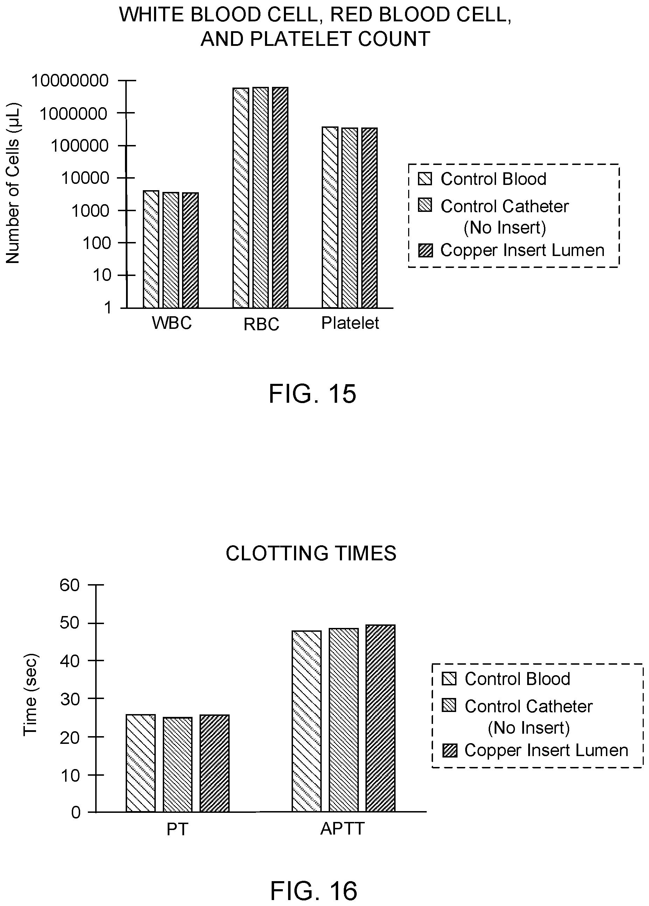

To evaluate the safety and biological factors related to exposing human blood to a 20 cm copper wire in a hemodialysis catheter, the studies shown in FIGS. 15 and 16 were carried out to verify that the copper wire would have no impact on blood cell count and blood clotting times, which are two metrics used to evaluate healthy blood.

As can be seen in FIG. 15, there was virtually no difference in white blood count, red blood count, or platelet count when comparing a control blood sample, a luminal blood sample without any inserts or coatings present, and a luminal blood sample with the 20 cm copper wire therein. FIG. 16 illustrates that the clotting times are also virtually unaffected compared to the respective controls as described above, thus indicating that contact with copper wire does not impact the function of the blood.

While the forgoing example and description is illustrative of the principles of the present technology in one or more particular applications, it will be apparent to those of ordinary skill in the art that numerous modifications in form, usage and details of implementation can be made without the exercise of inventive faculty, and without departing from the principles and concepts of this technology. Accordingly, it is not intended that the technology be unduly limited.

## Claims

1. A tube-containing medical device kit, comprising:
a tube-containing medical device (110) including an access opening (112) positionable exterior with respect to a subject, a fluid communication opening (118) positionable within the subject to fluidically communicate with a body fluid of the subject, and an elongated lumen (120) therebetween; and
a luminal metal wire (130) insertable within the elongated lumen (120), wherein the luminal metal wire (130) has a length that is within 5% to 100% of the elongated lumen (120), and has an x-y cross-sectional diameter sufficiently smaller than an x-y cross-sectional diameter of the elongated lumen (120) to allow fluid flow therebetween, wherein the luminal metal wire (130) comprises an antimicrobial wire substrate that is uncoated or is partially coated with a bioactive agent leaving some antimicrobial wire substrate portions exposed, and wherein an x-y-cross-sectional area of the luminal metal wire (130) occupies an average of 7% to 90% of the x-y-dimension cross-section area of the elongated lumen (120) at locations where the luminal metal wire (130) is fully inserted or insertable within the elongated lumen (120).

2. The tube-containing medical device kit of claim 1, wherein:
the luminal metal wire (130) extends at least from the access opening (112) and through the elongated lumen (120) such that a distal tip (132) of the luminal metal wire (130) is positioned short of the fluid communication opening (118) by a distance of 0.1 cm to 55 cm; or
the tube-containing medical device is a catheter with the fluid communication opening positioned on a side wall of the elongated lumen, wherein the luminal metal wire (130) is of a length so that a distal tip (132) of the luminal metal wire (130) extends beyond the fluid communication opening but still remains within the elongated lumen.

3. The tube-containing medical device kit of claim 1, wherein the access opening (112) includes a hub connector (114).

4. The tube-containing medical device kit of claim 1, wherein the luminal metal wire (130) is a solid metal or metal alloy wire of copper, silver, zinc, gold, tin, or an alloy thereof.

5. The tube-containing medical device kit of claim 1, wherein the luminal metal wire (130) comprises copper or a copper alloy, and wherein the luminal metal wire is a contact killer of microbial organisms.

6. The tube-containing medical device kit of claim 1, wherein the tube-containing medical device kit is assembled in the form of a tube-containing medical device assembly (100) with the luminal metal wire (130) inserted within the elongated lumen (120).

7. The tube-containing medical device kit of claim 1, wherein the bioactive agent includes an antimicrobial agent, anti-thrombogenic agent, a thrombolytic agent, a drug, or a combination thereof.

8. The tube-containing medical device kit of claim 1, wherein:
the luminal metal wire (130) has a z-dimension length to x-y-dimension cross-sectional diameter ratio of 50:1 to 3,000:1.

9. The tube-containing medical device kit of claim 1, wherein the luminal metal wire includes:
multiple metal wires that are braided, twisted, composited, or soldered together;
surface texture; or
an open core with a metal or metal alloy shell that is antimicrobial, wherein the core is loaded with bioactive agent and the shell includes pores or includes an orifice at an end of the luminal metal wire for releasing the bioactive agent therefrom.

10. The tube-containing medical device kit of claim 1, wherein the tube-containing medical device is a urinary catheter, a vascular catheter, a shunt, a drain, a tracheal tube, or a feeding tube.

11. The tube-containing medical device kit of claim 1, further comprising a fluid lock solution for loading in the elongated lumen.

12. The tube-containing medical device kit of claim 1, wherein the tube-containing medical device includes a hub opening for inserting or removing the luminal metal wire (130) from the elongated lumen (120) that is separate from a fluidic hub opening used for introducing or removing fluid from the elongated lumen (120).

13. The tube-containing medical device kit of claim 1, where the luminal metal wire (130) includes a proximal end and a distal tip (132), wherein the luminal metal wire includes both an antimicrobial agent and anti-thrombogenic agent coated thereon, and wherein the antimicrobial agent is nearer to the proximal end and the anti-thrombogenic agent is nearer the distal tip (132).

14. A method of making the tube-containing medical device kit of any one of the preceding claims, comprising:
obtaining the tube-containing medical device (110);
establishing a length of the luminal metal wire (130) that is positionable within 5% to 100% of the elongated lumen (120), wherein the luminal metal wire (130) has an x-y cross-sectional diameter sufficiently smaller than an x-y cross-sectional diameter of the elongated lumen (120) to allow insertion and removal of the luminal metal wire from the elongated lumen; and
sizing the luminal metal wire (130) to correspond with the length so that when inserted or after insertion within the elongated lumen (120), the luminal metal wire does not protrude out from the fluid communication opening (118).

15. The tube-containing medical device kit of claim 1, wherein the luminal metal wire (130) has a length that is within 50% to 100% of the elongated lumen (120).

## Patentansprüche

1. Kit eines medizinischen Geräts, das eine Röhre enthält, umfassend:
ein medizinisches Gerät (110), das eine Röhre enthält, welches eine mit Bezug auf einen Patienten außen positionierbare Zugangsöffnung (112), eine zur fluidischen Kommunikation mit einer Körperflüssigkeit des Patienten innerhalb des Patienten positionierbare Fluidkommunikationsöffnung (118) und ein längliches Lumen (120) dazwischen aufweist; und
einen innerhalb des länglichen Lumens (120) einführbaren luminalen Metalldraht (130), wobei der luminale Metalldraht (130) eine Länge aufweist, die innerhalb von 5 % bis 100 % des länglichen Lumens (120) liegt, und einen x-y-Querschnittsdurchmesser aufweist, der hinreichend kleiner als ein x-y-Querschnittsdurchmesser des länglichen Lumens (120) ist, um einen Flüssigkeitsfluss dazwischen zuzulassen, wobei der luminale Metalldraht (130) ein antimikrobielles Drahtsubstrat umfasst, das unbeschichtet oder teilweise mit einem bioaktiven Mittel beschichtet ist, wodurch einige Abschnitte des antimikrobiellen Drahtsubstrats weiter freiliegen, und wobei eine x-y-Querschnittsfläche des luminalen Metalldrahts (130) durchschnittlich 7 % bis 90 % der x-y-Dimensions-Querschnittsfläche des länglichen Lumens (120) an Stellen, an denen der luminale Metalldraht (130) ganz innerhalb des länglichen Lumens (120) eingeführt wird oder einführbar ist, einnimmt.

2. Kit eines medizinischen Geräts, das eine Röhre enthält, nach Anspruch 1, wobei:
der luminale Metalldraht (130) sich mindestens von der Zugangsöffnung (112) und durch das längliche Lumen (120) erstreckt, sodass eine distale Spitze (132) des luminalen Metalldrahts (130) um einen Abstand von 0,1 cm bis 55 cm von der Fluidkommunikationsöffnung (118) entfernt positioniert ist; oder
das medizinische Gerät, das eine Röhre enthält, ein Katheter ist, dessen Fluidkommunikationsöffnung auf einer Seitenwand des länglichen Lumens positioniert ist, wobei der luminale Metalldraht (130) so lang ist, dass sich eine distale Spitze (132) des luminalen Metalldrahts (130) über die Fluidkommunikationsöffnung hinaus erstreckt, jedoch weiter innerhalb des länglichen Lumens bleibt.

3. Kit eines medizinischen Geräts, das eine Röhre enthält, nach Anspruch 1, wobei die Zugangsöffnung (112) ein Hubanschlussstück (114) aufweist.

4. Kit eines medizinischen Geräts, das eine Röhre enthält, nach Anspruch 1, wobei der luminale Metalldraht (130) ein massiver Metall- oder Metalllegierungsdraht aus Kupfer, Silber, Zink, Gold, Zinn oder einer Legierung davon ist.

5. Kit eines medizinischen Geräts, das eine Röhre enthält, nach Anspruch 1, wobei der luminale Metalldraht (130) Kupfer oder eine Kupferlegierung umfasst und wobei der luminale Metalldraht mikrobielle Organismen durch Kontaktabtötung abtötet.

6. Kit eines medizinischen Geräts, das eine Röhre enthält, nach Anspruch 1, wobei das Kit eines medizinischen Geräts, das eine Röhre enthält, in Form einer Baugruppe (100) eines medizinischen Geräts, das eine Röhre enthält, mit einem innerhalb des länglichen Lumens (120) eingeführten luminalen Metalldraht (130) zusammengesetzt ist.

7. Kit eines medizinischen Geräts, das eine Röhre enthält, nach Anspruch 1, wobei das bioaktive Mittel ein Antimikrobiotikum, ein Antithrombotikum, ein Thrombolytikum, ein Medikament oder eine Kombination davon beinhaltet.

8. Kit eines medizinischen Geräts, das eine Röhre enthält, nach Anspruch 1, wobei:
der luminale Metalldraht (130) ein Verhältnis von 50:1 zu 3000:1 einer z-Dimensions-Länge zu einem x-y-Dimensions-Querschnittsdurchmesser aufweist.

9. Kit eines medizinischen Geräts, das eine Röhre enthält, nach Anspruch 1, wobei der luminale Metalldraht Folgendes aufweist:
mehrere Metalldrähte, die umflochten, verdrillt, zusammengesetzt oder
zusammengelötet sind;
eine Oberflächentextur; oder
einen offenen Kern mit einem Metall- oder Metalllegierungsmantel, der antimikrobiell ist, wobei der Kern mit einem bioaktiven Mittel befüllt ist und der Mantel Poren aufweist oder eine Austrittsöffnung an einem Ende des luminalen Metalldrahts zum Freisetzen des bioaktiven Mittels daraus aufweist.

10. Kit eines medizinischen Geräts, das eine Röhre enthält, nach Anspruch 1, wobei das medizinische Gerät, das eine Röhre enthält, ein Blasenkatheter, ein Gefäßkatheter, ein Shunt, ein Drain, ein Trachealtubus oder eine Ernährungssonde ist.

11. Kit eines medizinischen Geräts, das eine Röhre enthält, nach Anspruch 1, ferner umfassend eine Flüssigkeitsverschlusslösung zum Befüllen in dem länglichen Lumen.

12. Kit eines medizinischen Geräts, das eine Röhre enthält, nach Anspruch 1, wobei das medizinische Gerät, das eine Röhre enthält, eine Huböffnung zum Einführen oder Entfernen des luminalen Metalldrahts (130) aus dem länglichen Lumen (120) aufweist, die getrennt von einer fluidischen Huböffnung ist, die zum Einführen oder Entfernen von Fluid aus dem länglichen Lumen (120) verwendet wird.

13. Kit eines medizinischen Geräts, das eine Röhre enthält, nach Anspruch 1, wobei der luminale Metalldraht (130) ein proximales Ende und eine distale Spitze (132) aufweist, wobei der luminale Metalldraht sowohl mit einem Antimikrobiotikum als auch mit einem Antithrombotikum beschichtet ist und wobei das Antimikrobiotikum näher bei dem proximalen Ende ist und das Antithrombotikum näher bei der distalen Spitze (132) ist.

14. Verfahren zum Herstellen des Kits eines medizinischen Geräts, das eine Röhre enthält, nach einem der vorhergehenden Ansprüche, umfassend:
Erhalten des medizinischen Geräts (110), das eine Röhre enthält;
Festlegen einer Länge des luminalen Metalldrahts (130), der innerhalb von 5 % bis 100 % des länglichen Lumens (120) liegt, wobei der luminale Metalldraht (130) einen x-y-Querschnittsdurchmesser aufweist, der hinreichend kleiner als ein x-y-Querschnittsdurchmesser des länglichen Lumens (120) ist, um eine Einführung und Entfernung des luminalen Metalldrahts aus dem länglichen Lumen zuzulassen; und
Festlegen der Größe des luminalen Metalldrahts (130), sodass sie mit der Länge korrespondiert, damit, sobald er eingeführt wird oder nach der Einführung innerhalb des länglichen Lumens (120), der luminale Metalldraht nicht aus der Fluidkommunikationsöffnung (118) herausragt.

15. Kit eines medizinischen Geräts, das eine Röhre enthält, nach Anspruch 1, wobei der luminale Metalldraht (130) eine Länge aufweist, die innerhalb von 50 % bis 100 % des länglichen Lumens (120) liegt.

## Revendications

1. Kit de dispositif médical contenant un tube, comprenant :
un dispositif médical contenant un tube (110) comprenant une ouverture d'accès (112) pouvant être positionnée à l'extérieur par rapport à un sujet, une ouverture de communication fluidique (118) pouvant être positionnée à l'intérieur du sujet pour communiquer fluidiquement avec un fluide corporel du sujet, et une lumière allongée (120) entre celles-ci ; et
un fil métallique luminal (130) pouvant être inséré dans la lumière allongée (120), le fil métallique luminal (130) ayant une longueur qui est comprise entre 5 % et 100 % de la lumière allongée (120) et ayant un diamètre transversal x-y suffisamment plus petit qu'un diamètre transversal x-y de la lumière allongée (120) pour permettre l'écoulement de fluide entre eux, le fil métallique luminal (130) comprenant un substrat de fil antimicrobien qui n'est pas enduit ou qui est partiellement enduit d'un agent bioactif laissant certaines parties du substrat de fil antimicrobien exposées, et une surface transversale x-y du fil métallique luminal (130) occupant en moyenne 7 % à 90 % de la surface transversale selon la dimension x-y de la lumière allongée (120) à des emplacements où le fil métallique luminal (130) est ou peut être entièrement inséré dans la lumière allongée (120).

2. Kit de dispositif médical contenant un tube selon la revendication 1,
le fil métallique luminal (130) s'étendant au moins depuis l'ouverture d'accès (112) et dans la lumière allongée (120) de telle sorte qu'une pointe distale (132) du fil métallique luminal (130) soit positionnée à proximité de l'ouverture de communication fluidique (118) à une distance comprise entre 0,1 cm et 55 cm ; ou
le dispositif médical contenant un tube étant un cathéter dont l'ouverture de communication fluidique est positionnée sur une paroi latérale de la lumière allongée, le fil métallique luminal (130) ayant une longueur telle qu'une extrémité distale (132) du fil métallique luminal (130) s'étend au-delà de l'ouverture de communication fluidique mais reste néanmoins à l'intérieur de la lumière allongée.

3. Kit de dispositif médical contenant un tube selon la revendication 1, l'ouverture d'accès (112) comprenant un raccord de moyeu (114).

4. Kit de dispositif médical contenant un tube selon la revendication 1, le fil métallique luminal (130) étant un fil solide en métal ou en alliage métallique composé de cuivre, d'argent, de zinc, d'or, d'étain ou d'un alliage de ceux-ci.

5. Kit de dispositif médical contenant un tube selon la revendication 1, le fil métallique luminal (130) comprenant du cuivre ou un alliage de cuivre, et le fil métallique luminal étant un agent destructeur de micro-organismes par contact.

6. Kit de dispositif médical contenant un tube selon la revendication 1, le kit de dispositif médical contenant un tube étant assemblé sous la forme d'un ensemble dispositif médical contenant un tube (100) avec le fil métallique luminal (130) inséré dans la lumière allongée (120).

7. Kit de dispositif médical contenant un tube selon la revendication 1, l'agent bioactif comprenant un agent antimicrobien, un agent antithrombogène, un agent thrombolytique, un médicament ou une combinaison de ceux-ci.

8. Kit de dispositif médical contenant un tube selon la revendication 1,
le fil métallique luminal (130) présentant un rapport entre la longueur selon la dimension z et le diamètre transversal selon la dimension x-y compris entre 50:1 et 3 000:1.

9. Kit de dispositif médical contenant un tube selon la revendication 1, le fil métallique luminal comprenant :
de multiples fils métalliques qui sont tressés, torsadés, regroupés ou soudés ensemble ; une texture de surface ; ou
une âme ouverte avec une enveloppe en métal ou en alliage métallique qui est antimicrobienne, l'âme étant chargée d'un agent bioactif et l'enveloppe comprenant des pores ou comprenant un orifice au niveau d'une extrémité du fil métallique luminal pour libérer l'agent bioactif par celui-ci.

10. Kit de dispositif médical contenant un tube selon la revendication 1, le dispositif médical contenant un tube étant un cathéter urinaire, un cathéter vasculaire, un shunt, un drain, un tube trachéal ou une sonde d'alimentation.

11. Kit de dispositif médical contenant un tube selon la revendication 1, comprenant en outre une solution de verrouillage de fluide destinée à être chargée dans la lumière allongée.

12. Kit de dispositif médical contenant un tube selon la revendication 1, le dispositif médical contenant un tube comprenant une ouverture de moyeu qui permet l'insertion ou le retrait du fil métallique luminal (130) de la lumière allongée (120) et qui est séparée d'une ouverture de moyeu fluidique utilisée permettant l'introduction ou le retrait d'un fluide de la lumière allongée (120).

13. Kit de dispositif médical contenant un tube selon la revendication 1, le fil métallique luminal (130) comprenant une extrémité proximale et une pointe distale (132), le fil métallique luminal étant enduit à la fois d'un agent antimicrobien et d'un agent antithrombogène, et l'agent antimicrobien étant plus proche de l'extrémité proximale et l'agent antithrombogène étant plus proche de la pointe distale (132).

14. Procédé de fabrication du kit de dispositif médical contenant un tube selon l'une quelconque des revendications précédentes, consistant à :
obtenir le dispositif médical contenant le tube (110) ;
établir une longueur du fil métallique luminal (130) qui peut être positionnée entre 5 % et 100 % de la lumière allongée (120), le fil métallique luminal (130) ayant un diamètre transversal x-y suffisamment plus petit qu'un diamètre transversal x-y de la lumière allongée (120) pour permettre l'insertion et le retrait du fil métallique luminal de la lumière allongée ; et
dimensionner le fil métallique luminal (130) pour qu'il corresponde à la longueur, de telle sorte que, lorsqu'il est inséré ou après son insertion dans la lumière allongée (120), le fil métallique luminal ne dépasse pas de l'ouverture de communication fluidique (118).

15. Kit de dispositif médical contenant un tube selon la revendication 1, le fil métallique luminal (130) ayant une longueur qui est comprise entre 50 % et 100 % de la lumière allongée (120).
